# EUROPEAN PATENT APPLICATION

(11) **EP 1 312 381 A1**
(43) Date of publication of application: **21.05.2003**
(21) Application number: 01941167.7
(22) Date of filing: 21.06.2001
(51) Int. Cl.: A61K 51/12, A61K 33/10, A61K 9/00, A61K 47/32, A61K 47/38

(54) **PREPARATIONS FOR MEASRUING GASTRIC pH VALUE AND METHOD OF MEASURING GASTRIC pH VALUE BY USING THE SAME**

(30) Priority: 21.06.2000 JP 2000186589
(71) Applicant: OTSUKA PHARMACEUTICAL CO., LTD., Chiyoda-ku Tokyo 101-8535 (JP)
(72) Inventor: INADA, Makoto, Itano-gun, Tokushima 771-0205 (JP); NODA, Atsunari, Itano-gun, Tokushima 779-0104 (JP); IKEI, Nobuhiro, Kawauchi-cho, Tokushima 771-0136 (JP)
(74) Representative: HOFFMANN - EITLE
(86) International application number: JP0105331
(87) International publication number: WO01097863

(57) **Abstract**

The present invention provides a preparation for easily and non-invasively measuring gastric pH, for example, a preparation for measuring gastric pH using the expired air, and a method for measuring gastric pH using the preparation. The preparation of the present invention can be produced by covering a composition containing a compound labeled with an isotope (¹³C, ¹⁴C, ¹⁵N, or ¹⁸O), with a pH-dependent soluble base.

## Description

### TECHNICAL FIELD

The present invention relates to a technique for measuring gastric pH, and specifically a preparation for measuring gastric pH and a method for measuring gastric pH using the preparation. More specifically, the present invention relates to a preparation for non-invasively measuring gastric pH using the expired air, and a method for measuring gastric pH using the preparation.

### BACKGROUND ART

A large number of medicines are synthesized in the form of organic acids or organic bases. It is known that some of these organic acids and organic bases are influenced by gastric pH, causing large changes in bioavailability, and as a result they do not produce the expected pharmacological effects or cause unexpected and severe side effects. Further, in today's aging society, the number of patients with hypoacidity or anacidity is said to be rapidly increasing.

In the case of such patients, it is believed that measuring the gastric pH tendency (hyperacidity, normal, hypoacidity, or anacidity) before medication provides very useful information for predicting the therapeutic and side effects of the medicine.

The advent of H2-antagonists and gastric acid secretion inhibitors (proton pump inhibitors: PPIs) has greatly contributed to the treatment of gastric and duodenal ulcers. However, recrudescence or recurrence (in particular recurrence of reflux esophagitis) after treatment has become a problem in recent years, and thus medicinal treatment methods for gastric and duodenal ulcers, including revisions of treatment methods, are attracting the attention as a subject to be examined in the medical field. Treatment with an H2-antagonist or PPI suppresses gastric acid secretion, and its therapeutic effect is assessed by using the increase in gastric pH as an index. The recurrence of reflux esophagitis is a rebound phenomenon caused when the administration of an H2 antagonist, PPI, or the like medicine is discontinued, and is presumed to be predictable to some extent by measuring the gastric pH.

Thus, the measurement of gastric pH presumably makes it possible to predict the therapeutic and side effects of a medicine to some extent. Further, it is believed that the measurement of gastric pH will find wide application in evaluating therapeutic effects and diagnosing diseases.

Known methods for measuring gastric pH include: a method comprising administering to a subject a preparation obtained by filling gastro-soluble capsules with vitamin B2, and then measuring the concentration of vitamin B2 excreted in the urine by HPLC (GA-Test: J. Pharm. Dyn., 7, 656-664 (1984)); and a method comprising inserting an endoscope having pH measuring electrodes at the end into a subject's stomach to directly measure gastric pH (Digestive Disease and Sciences, Vol. 42, No.11 2304-2309 (1997)). However, the first method is incapable of accurately measuring gastric pH because of the influence of other factors, such as metabolism. The second method is defective in that it causes pain to the subject since the endoscope is directly inserted into the stomach.

Thus, there is no known method for easily measuring gastric pH with high accuracy and reproducibility and without hurting or placing any other burdens on the subject.

### DISCLOSURE OF THE INVENTION

An object of the present invention is to provide a preparation for easily and non-invasively measuring gastric pH. More specifically, the present invention aims to provide a preparation for measuring and assessing gastric pH using the expired air. Another object of the present invention is to provide a method for easily measuring gastric pH using the preparation.

In view of the status quo, the present inventors conducted extensive research to solve the above problems. As a result, the present inventors found that when a ¹³C-labeled compound in the form of a preparation that dissolves depending on pH is orally administered to a subject, such as a human, the behavior of the ¹³C-labeled compound excreted from the body changes according to gastric pH, and that there is a constant relationship between gastric pH and the excretion behavior of the labeled compound. Further, the present inventors confirmed that the gastric pH tendency can be measured and assessed by measuring the in vivo or excretion behavior of the ¹³C-labeled compound.

The present invention has been accomplished based on these findings.

The present invention provides the preparations for measuring gastric pH described in the following items 1 to 9:
1. A preparation for measuring gastric pH comprising a composition containing an isotope-labeled compound, and a pH-dependent soluble base covering the composition.
2. A preparation according to item 1, wherein the isotope is at least one member selected from the group consisting of ¹³C, ¹⁴C, ¹⁵N, and ¹⁸O.
3. A preparation according to item 1 or 2, wherein the isotope-labeled compound is an alkali metal carbonate, an alkaline earth metal carbonate, ammonium carbonate, an alkali metal hydrogencarbonate, or ammonium hydrogencarbonate.
4. A preparation according to item 3, wherein the isotope-labeled compound is at least one member selected from the group consisting of sodium carbonate, potassium carbonate, calcium carbonate, magnesium carbonate, ammonium carbonate, potassium hydrogencarbonate, sodium hydrogencarbonate, and ammonium hydrogencarbonate.
5. A preparation according to any one of items 1 to 4, wherein the composition containing an isotope-labeled compound further contains at least one acid compound selected from the group consisting of citric acid, tartaric acid, and malic acid.
6. A preparation according to item 1, wherein the isotope-labeled compound is at least one member selected from the group consisting of amino acids, proteins, organic acids, organic acid salts, saccharides, and lipids.
7. A preparation according to any one of items 1 to 6, wherein the pH-dependent soluble base is an enteric base or gastro-soluble base.
8. A preparation according to item 7, wherein the pH-dependent soluble base is at least one gastro-soluble base selected from the group consisting of methyl methacrylate-butyl methacrylate-dimethylaminoethyl methacrylate copolymers, and polyvinyl acetal diethyl acetate.
9. A preparation according to item 7, wherein the pH-dependent soluble base is at least one enteric base selected from the group consisting of hydroxypropylmethylcellulose phthalate, methacrylic acid-methyl methacrylate copolymers, hydroxypropylmethylcellulose acetate succinate, carboxymethylethylcellulose, hydroxypropylmethylcellulose phthalate, and cellulose acetate phthalate.
   These preparations are in oral dosage forms, such as tablets, capsules, pills, powders, and granules.
   The present invention further provides a method for measuring gastric pH using any one of the above preparations, specifically the methods described in the following items 10 to 14. .
10. A method for measuring gastric pH using a preparation according to any one of items 1 to 9.
11. A method for measuring gastric pH, comprising orally administering a preparation according to item 1 to a subject, and measuring the behavior of a labeled compound excreted from the body.
12. A method for measuring gastric pH, comprising orally administering a preparation according to item 2 to a subject, and measuring the amount or behavior of labeled CO₂ excreted in the expired air.
13. A method according to item 10, wherein a preparation according to any one of items 1 to 7 and 9 comprising an enteric base as the pH-dependent soluble base is used in combination with a preparation according to any one of items 1 to 8 comprising a gastro-soluble base as the pH-dependent soluble base.
14. A method for measuring gastric pH in a subject, comprising orally administering a preparation according to any one of items 1 to 9 to a subject who is suspected of having a decreased or increased gastric pH, and comparing the behavior of a labeled compound excreted from the body after a prescribed time withy a standard control.
   The present invention also provides use of any one of the above preparations for measuring gastric pH.
15. Use of a preparation according to any one of items 1 to 9 in a method for measuring gastric pH.
16. Use of an isotope-labeled compound covered with a pH-dependent soluble base, for producing a preparation for measuring gastric pH for use in a method for measuring gastric pH.
17. Use according to item 16, wherein the isotope-labeled compound is an alkali metal carbonate, an alkaline earth metal carbonate, ammonium carbonate, an alkali metal hydrogencarbonate, or ammonium hydrogencarbonate.

The gastric pH measurement according to the present invention is not limited to the measurement of a specific gastric pH value, but broadly includes the measurement of the gastric pH tendency for determining a variety of cases relating to gastric pH, such as hyperacidity, normal, hypoacidity, and anacidity.

### BRIEF DESCRIPTION OF THE DRAWINGS

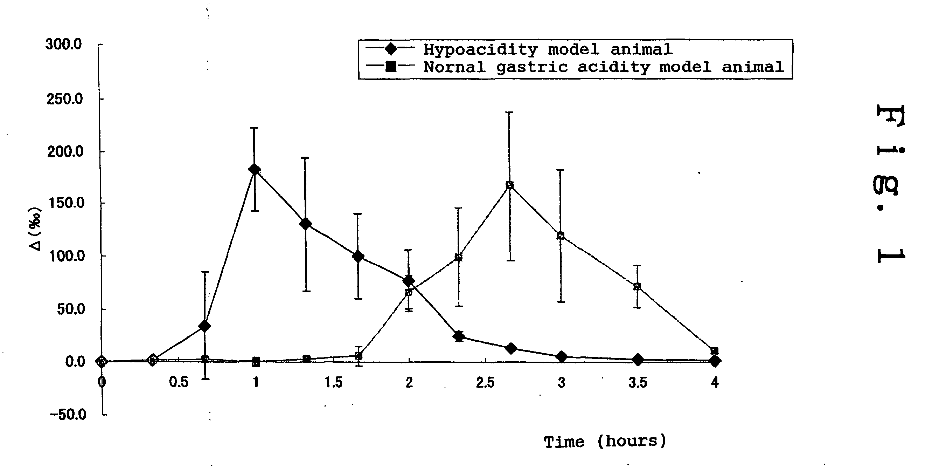

Fig. 1 shows the results of Example 1, in which enteric capsules of the present invention were administered to hypoacidity model animals (-◆-) and normal acidity model animals (-■-), and the difference (Δ(‰)) in the carbon dioxide ¹³CO₂/¹²CO₂ ratio in the expired air between before and after the administration of the enteric capsules was sequentially traced over time.

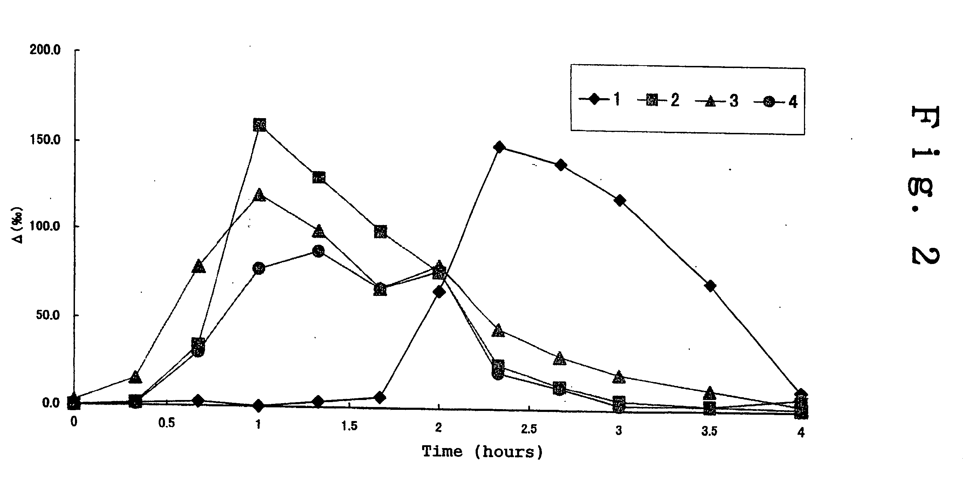

Fig. 2 shows the results of Example 2, in which enteric capsules of the present invention were administered to experimental animals (n=4), and the difference (Δ(‰)) in the carbon dioxide ¹³CO₂/¹²CO₂ ratio in the expired air of the animals (Nos. 1 to 4) between before and after the administration of the enteric capsules was sequentially traced over time.

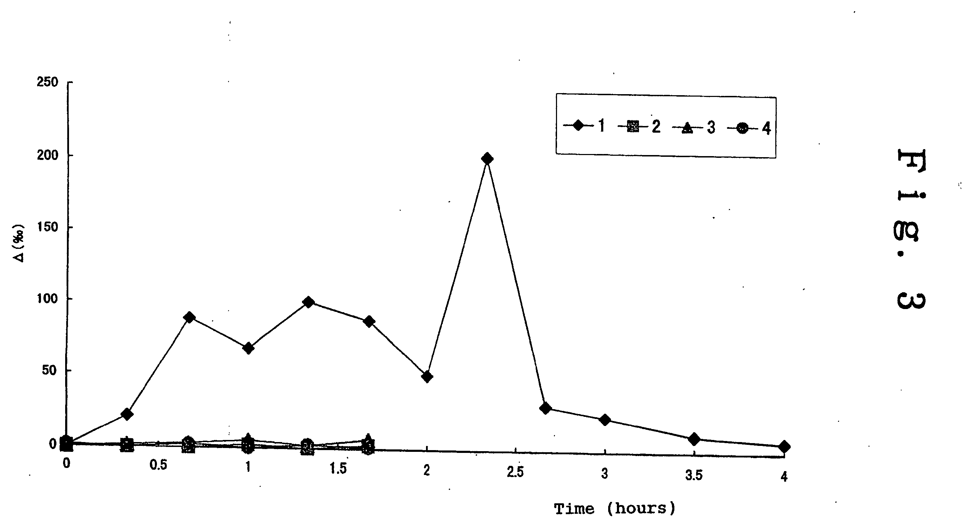

Fig. 3 shows the results of Example 2, in which gastro-soluble capsules of the present invention were administered to experimental animals (n=4), and the difference (Δ (‰)) in the carbon dioxide ¹³CO₂/¹²CO₂ ratio in the expired air of these animals (Nos. 1 to 4) between before and after the administration of the gastro-soluble capsules was sequentially traced over time.

### BEST MODE FOR CARRYING OUT THE INVENTION

### (1) Preparation for measuring gastric pH

The preparation for measuring gastric pH of the present invention is characterized by comprising a composition containing an isotope-labeled compound, and a pH-dependent soluble base covering the composition.

The isotope-labeled compound is not limited and may be any compound that, after being orally administered to a subject, dissolves and in some cases is degraded or metabolized in the subject's body, and excreted in the expired air or a body fluid (urine, blood, saliva, sweat, or the like).

A preferred example of the isotope-labeled compound is a compound that rapidly appears as carbon dioxide (CO₂) in the expired air, after being dissolved and in some cases degraded and metabolized in the body.

Examples of compounds that rapidly appear as carbon dioxide in the expired air after being dissolved in the body include a wide variety of compounds that generate carbonate ions (CO₃⁻²) or hydrogencarbonate ions (HCO₃⁻¹) when dissolved. Examples of such compounds include alkali metal salts of carbonic acid, such as sodium carbonate and potassium carbonate; alkaline earth metal salts of carbonic acid, such as calcium carbonate and magnesium carbonate; ammonium carbonate; alkali metal hydrogencarbonate, such as potassium hydrogencarbonate and sodium hydrogencarbonate; ammonium hydrogencarbonate; and the like. These compounds, when used in combination with an acid compound, such as citric acid, tartaric acid, or malic acid, rapidly appear as carbon dioxide in the expired air. Preferably, sodium carbonate, potassium carbonate, sodium hydrogencarbonate, or potassium hydrogencarbonate is used.

Examples of compounds that appear as carbon dioxide in the expired air after being dissolved and then degraded or metabolized in the body include amino acids, proteins, organic acids, salts (e.g., alkali metal salts, such as Na) of organic acids, saccharides, lipids, and the like. These compounds generate carbon dioxide in the expired air via the hepatic metabolism, after being digested and absorbed. Examples of amino acids include glycine, phenylalanine, tryptophan, methionine, valine, histidine, and the like. Examples of organic acids include acetic acid, lactic acid, pyruvic acid, butyric acid, propionic acid, octanoic acid, and their alkali metal salts. Examples of saccharides include glucose, galactose, xylose, lactose, and the like. Examples of lipids include medium chain triglycerides, such as trioctanoin. However, these examples are not limitative. Preferably, an amino acid, such as glycine, an organic acid, such as acetic acid or octanoic acid, or an alkali metal salt (e.g., sodium salt or potassium salt) of such an organic acid can be used.

Examples of isotopes used to label these compounds include ¹³C, ¹⁴C, ¹⁵N, ¹⁸O, and the like. The isotopes may be radioactive or non-radioactive, but is preferably non-radioactive from the safety point of view. Such isotopes include ¹³C, ¹⁵N, ¹⁸O, and the like, and ¹³C can be mentioned as a suitable example.

The method for labeling with these isotopes is not limited and may be a conventional one. Further, a wide variety of known or commercially available compounds labeled with these isotopes are usable (Sasaki, "5.1 Application of Stable Isotopes in Clinical Diagnosis": Kagaku no Ryoiki (Journal of Japanese Chemistry) 107, "Application of Stable Isotopes in Medicine, Pharmacy, and Biology", pp. 149-163 (1975), Nankodo: Kajiwara, RADIOISOTOPES, 41, 45-48 (1992), etc.).

The preparation for measuring gastric pH of the present invention is produced by covering the above isotope-labeled compound or a composition containing the compound with a pH-dependent soluble base.

The compound to be covered with the pH-dependent soluble base may be used singly, or may be used in the form of a composition prepared by adding, as other ingredients, for example, an excipient, such as lactose, sucrose, sodium chloride, glucose, urea, starch, calcium carbonate, kaolin, crystalline cellulose, or silicic acid; a binder, such as simple syrup, glucose solution, starch solution, gelatin solution, carboxymethylcellulose, shellac, methylcellulose, potassium phosphate, or polyvinyl pyrrolidone; a disintegrator, such as dry starch, sodium alginate, agar powder, laminaran powder, polyoxyethylene sorbitan fatty acid esters, sodium lauryl sulfate, stearic acid monoglyceride, starch, or lactose; an absorption accelerator, such as quaternary ammonium base or sodium lauryl sulfate; a humectant, such as glycerin or starch; a lubricant, such as purified talc, stearate, boric acid powder, or polyethylene glycol; other additives (for example, a flavor improver, taste improver, stabilizer, etc.); or the like.

The mode of covering with the pH-dependent soluble base is not limited, as long as the preparation for measuring gastric pH is designed so that, after the orally administering the preparation to a subject, the base dissolves first depending on the pH in the subject's body, and the isotope-labeled compound dissolves during or after the dissolution of the base. Examples of this mode include a coated preparation that the surface of an isotope-labeled compound or a composition containing the compound (hereinafter collectively referred to as a "labeled compound-containing material"), which is prepared in form of particles, granules, tablets, pills, or the like, is covered with a pH-dependent soluble base; and a capsule preparation that a labeled compound-containing material prepared in form of particles, granules, liquid, semisolid, or the like, is encapsulated with a pH-dependent soluble capsule base. These modes of covering can be carried out in a routine manner.

The pH-dependent soluble base for use in the present invention may be a gastro-soluble or enteric base.

Gastro-soluble bases are bases that dissolve under acidic conditions that generally occur at the gastric pH in a healthy person or a patient with hyperacidity, specifically at pH 5 or lower. Specific examples include acid-soluble polymers, such as aminoacryl methacrylate copolymer E (tradename: Eudragit E100, manufactured by Rohm Pharma) and like methyl methacrylate-butyl methacrylate-dimethylaminoethyl methacrylate copolymers, and polyvinyl acetal diethyl acetate (tradename: AEA, manufactured by Sankyo Co., Ltd.).

Enteric bases are bases that dissolve under weakly alkaline conditions that generally occur at the gastric pH in a patient with hypoacidity or anacidity, specifically at pH 5 or higher. Specific examples include enteric polymers, such as hydroxypropylmethylcellulose phthalate (tradename: HP-55 or HP-50, manufactured by Shin-Etsu Chemical Co., Ltd.), methacrylic acid-methyl methacrylate copolymers (tradename: Eudragit S100, manufactured by Rohm Pharma) and like acrylic acid copolymers or methacrylic acid copolymers, hydroxypropylmethylcellulose acetate succinate (tradename: AQOAT, manufactured by Shin-Etsu Chemical Co., Ltd.), carboxymethylethylcellulose, hydroxypropylmethylcellulose phthalate, and cellulose acetate phthalate.

The preparation for measuring gastric pH of the present invention is designed so as to exhibit a dissolution behavior in accordance with the intended use. For example, when used for the diagnosis or evaluation of hyperacidity, the preparation is preferably produced using, as the pH-dependent soluble base, a gastro-soluble base that dissolves at pH 4 or lower. As a suitable example of such a gastro-soluble base, aminoacryl methacrylate copolymer E can be mentioned. This preparation is administered to a subject, and if labeled CO₂ gas is excreted in the subject's expired air more rapidly than the standard, i.e., the excretion behavior of labeled CO₂ gas in a healthy person's expired air, the subject is diagnosed as having hyperacidity.

When the preparation is used for diagnosing and evaluating hypoacidity or anacidity, it is desirable to produce the preparation using, as the pH-dependent soluble base, an enteric base that dissolves at pH 6 or higher. A suitable example of such an enteric base is hydroxypropylmethylcellulose phthalate (tradename: HP-55, manufactured by Shin-Etsu Chemical Co., Ltd.). This preparation is administered to a subject, and if labeled CO₂ gas appears in the subject's expired air more rapidly than the standard, i.e., the excretion behavior of labeled CO₂ gas in a healthy person's expired air, the subject is diagnosed as having hypoacidity or anacidity.

Gastric pH can be more correctly measured by using a combination of two preparations produced by covering a composition containing a compound labeled with a different isotope from each other with a base having a different pH dependency from each other.

For example, when a gastro-soluble preparation produced by covering, with a gastro-soluble base, a composition containing ¹³C-labeled compound (for example, NaH¹³CO₃) as the main ingredient, and an enteric preparation produced by covering, with an enteric base, a composition containing a trace amount of a ¹⁴C-labeled compound (for example, NaH¹⁴CO₃) are orally administered at the same time, only the ¹⁴C-labeled compound is excreted and detected outside the body or in a body fluid after a certain period of time in the case where the gastric pH has a hypoacidic tendency, and only the ¹³C-labeled compound is excreted and detected outside the body or in a body fluid after a certain period of time in the case where the gastric pH has a hyperacidic tendency. In the case where the gastric acidity is neutral, neither the ¹³C compound nor the ¹⁴C compound is detected.

Thus, using a combination of two preparations produced by covering a compound labeled with a different isotope from each other or a composition containing the compound with a base having a different pH dependency from each other, it is possible to specifically determine the gastric pH tendency (acidic, alkaline, or neutral) based on the difference in excretion behavior between the two isotope-labeled compounds (for example, the ¹³C compound and ¹⁴C compound).

Further, it is also possible to measure gastric pH by a combination of a urinary excretion test and an expiration test. Specifically stated, a gastro-soluble vitamin B₂ preparation and, for example, an enteric preparation containing ¹³C-labeled compound of the present invention are orally administered at the same time, and the vitamin B₂ concentration in the urine and the amount of ¹³CO₂ in the expired air are measured, to totally assess the gastric pH.

The form of the preparation for measuring gastric pH of the present invention is not limited as long as it is a solid form, and may be tablets, pills, granules, powder, or capsules. When the preparation is in the form of capsules, the form of the encapsulated pharmaceutical composition is not limited as long as the capsule base is a pH-dependent soluble base. The pharmaceutical composition may be a liquid, semiliquid, or solid (e.g., granules or powder).

The amount of the labeled compound-containing material in the preparation of the present invention is not limited, but can be suitably selected usually from the range of 1 to 5000 mg per unit dose, preferably from the range of 10 to 500 mg per unit dose.

The proportion of the isotope-labeled compound in the preparation of the present invention, although depending on the type of isotope to be used, is preferably 1 to 2000 mg, more preferably 10 to 200 mg, when the isotope is nonradioactive; and is preferably 0.1 to 10 µCi, more preferably 0.5 to 4 µCi, when the isotope is radioactive.

The proportion of the pH-dependent soluble base used for covering the labeled compound-containing material is, for example, 5 to 100 parts by weight, preferably 30 to 50 parts by weight, per 100 parts by weight of the labeled compound-containing material.

### (2) Method for measuring gastric pH

The present invention also provides a method for measuring gastric pH using the preparation for measuring gastric pH described above. Gastric pH can be measured by: orally administering the preparation of the present invention containing an isotope-labeled compound to a subject, such as an animal or human; collecting a biological sample, such as the expired air, urine, feces, blood, or another body fluid, preferably the expired air, urine, feces, or the like, more preferably the expired air; comparing the amount of a labeled substance excreted in the collected biological sample with the amount of the corresponding substance in a biological sample collected before the administration, to inspect the in vivo or excretion behavior of the substance; and comparing the behavior with a standard control. The standard control is not limited, and may be, for example, a standard obtained by finding a constant correlation between the in vivo or excretion behavior of the labeled substance measured by the method of the present invention and the gastric pH measured by a known gastric pH measuring method (GA-Test: J. Pharm. Dyn., 7, 656-664 (1984); Digestive Disease and Sciences, Vol. 42, No. 11 2304-2309 (1997)).

For example, when using the expired air as a biological sample and ¹³C as an isotope, the gastric pH tendency in a subject can be assessed in the following manner, according to a conventional ¹³C expiration test method (Kajiwara, RADIOISOTOPES, 41, 45-48 (1992); Kajiwara et al., RADIOISOTOPES, 41, 331-334 (1992), etc.): The preparation of the present invention is orally administered to the subject, and the expired air is sequentially collected over time. Then, the amount of ¹³CO₂ in the expired air, expressed as the ¹³CO₂/¹²CO₂ ratio (the δ¹³C value), is compared over time with the ¹³CO₂/¹²CO₂ ratio (the δ¹³C value) in the expired air before the administration, to measure the behavior of the ¹³CO₂ amount over time, and the behavior is compared with a standard control.

When the preparation of the present invention contains, as an isotope-labeled compound, a compound that dissolves and generates carbonate or hydrogencarbonate ions, such as a salt (an alkali metal salt, alkaline earth metal salt, ammonium salt, or the like) of carbonic acid, an alkali metal hydrogencarbonate, or ammonium hydrogencarbonate, gastric pH can be correctly reflected and measured, since the preparation is unlikely to be influenced by physiological factors, such as absorption and metabolism. Gastric pH can be assessed with higher accuracy by repeatedly using the preparation of the present invention several times, or by using a combination of two or more preparations each produced using, as the pH-dependent soluble base, a gastro-soluble or enteric base haying a different pH dependent solubility to compare the obtained results.

The labeled substance in the collected biological sample can be measured and analyzed by a conventional analysis technique, such as liquid scintillation counting, mass spectroscopy, infrared spectroscopic analysis, emission spectrochemical analysis, or nuclear magnetic resonance spectral analysis, which is selected depending on whether the isotope used for labeling is radioactive or non-radioactive. Infrared spectroscopic analysis and mass spectroscopy are preferable from the viewpoint of measurement accuracy.

The method and timing of administering the preparation of the present invention are not limited. Preferably, the preparation is administered on an empty stomach to avoid the influence of foods.

The amount of the isotope-labeled compound to be contained per unit dose of the preparation of the present invention varies depending on the test sample and the types of isotope and isotope-labeled compound to be used, and thus cannot be generally defined and is suitably adjusted and decided according to the case. For example, when gastric pH is measured by an expiration test using ¹³C-labeled sodium hydrogencarbonate (NaH¹³CO₂) as an isotope-labeled compound, it is desirable that the preparation contains 1 to 2000 mg, preferably 10 to 200 mg, of sodium hydrogencarbonate (NaH¹³CO₂) per unit dose.

When the preparation is administered to the body, the covering base (coating) on the surface of the preparation dissolves first by the influence of the pH in the body, and then the isotope-labeled compound inside the base begins to dissolve out. For example, when a preparation produced by covering a composition containing an isotope-labeled compound that rapidly appears as labeled carbon dioxide in the expired air after dissolution, such as NaH¹³CO₃, with a gastro-soluble base that usually dissolves at gastric pH, is administered to a subject, the labeled compound NaH¹³CO₃ dissolves out as the gastro-soluble base dissolves in the stomach, and labeled carbon-dioxide ¹³CO₂ is gradually excreted in the expired air as the labeled compound dissolves.

The ¹³CO₂ gas excreted in the expired air (expressed as, for example, the ratio of ¹³CO₂ to ¹²CO₂ (¹³CO₂/¹²CO₂)) shows a characteristic behavior according to the subject's gastric pH. For instance, when the above preparation, which comprises an gastro-soluble base, is used, the ¹³CO₂ gas excretion behavior in a subject with a decreased gastric pH, such as a patient with hyperacidity, tends to be more rapid than in a person with a normal gastric pH, and the ¹³CO₂ gas excretion behavior in a subject with an increased gastric pH, such as a patient with hypoacidity or anacidity, tends to be slower than in a person with a normal gastric pH.

When the preparation comprising an enteric base is used, the ¹³CO₂ gas excretion behavior in a subject with a decreased gastric pH, such as a patient with hyperacidity, tends to be slower than in a person with a normal gastric pH, and the ¹³CO₂ gas excretion behavior in a subject with an increased gastric pH, such as a patient with hypoacidity or anacidity, tends to be more rapid than in a person with a normal gastric pH.

Therefore, with the preparation for measuring gastric pH of the present invention, the presence or absence of a decrease or increase in gastric pH can be assessed by measuring the ¹³CO₂ gas amount in the expired air sequentially after orally administering the preparation, specifically, by measuring the carbon dioxide Δ value (‰) (the difference in the ¹³CO₂/¹²CO₂ concentration ratio (the δ¹³C value) between the expired air at each collection time after orally administering the preparation and the expired air before the administration). Thus, the method for measuring gastric pH of the present invention can also be defined as a method for diagnosing and assessing the presence or absence of a decrease or increase in gastric pH.

Further, when the labeled compound is, for example, an organic acid, such as acetic acid, or an amino acid, such as glycine, the compound discharged from the stomach is dissolved and absorbed in the intestines and metabolized in the liver, and then appears as labeled carbon dioxide in the expired air. Accordingly, the detection of a labeled substance (the excretion of labeled carbon dioxide ¹³CO₂ in the expired air) is slower than the above case. However, since the rate-limiting step is the dissolution of the covering base (coating) on a preparation, the use of such a compound also makes it possible to assess the presence or absence of a decrease/increase in gastric pH, like the use of the above-mentioned compounds, such as NaH¹³CO₃. When, for example, gastric pH is measured by an expiration test using acetic acid (CH₃¹³COOH) as a labeled compound, it is preferable that the preparation for measuring gastric pH of the present invention contains 1 to 2000 mg, preferably 10 to 200 mg, of acetic acid per unit dose.

The method for measuring gastric pH of the present invention is useful in that it can non-invasively and easily evaluate and diagnose gastric pH using a small number of expired air samples, without requiring the subject to spend a long time. Further, the method of the present invention makes it possible to assess the therapeutic effects for a disease relating to gastric pH, or the efficacy or therapeutic effects of a medicine relating to gastric pH. Specifically, gastric pH before and after administering a medicine to a subject is measured using the preparation for measuring gastric pH of the present invention, and the measured pH values are compared with each other. By this method, it is possible to assess the efficacies or the therapeutic effects of a medicine on a subject. As a result, the method can be used for selecting a medicine suitable for an individual subject. Examples of medicines relating to gastric pH include those that increase gastric pH, such as proton-pump inhibitors (PPIs) and H₂ blocker.

### EXAMPLES

The following examples are provided to illustrate the present invention in further detail, and are not to limit the scope of the present invention.

### Example 1

### (1) Production of a preparation for measuring gastric pH (capsules)

NaH¹³CO₃ (100 mg) was encapsulated in an enteric capsule base (60 mg; Capsule No. 1 defined in The Japanese Pharmacopoeia, hydroxypropylmethylcellulose phthalate, tradename: HP-55, manufactured by Shin-Etsu Chemical Co., Ltd. (dissolves at pH 5.5 or higher)), to produce an enteric preparation for measuring gastric pH (capsules). The following experiment was carried out using this preparation.

### (2) Animal experiment

The enteric capsules prepared above were administered to beagle dogs that had been artificially adjusted to have a normal gastric acidity or hypoacidity, and the ¹³CO₂ excretion behavior was measured. The detailed procedures of the experiment were as follows.

Four fasted beagle dogs were repeatedly used in the experiment.

To provide a normal gastric acidic condition to the beagle dogs, the expired air was collected and 20 ml of 0.1N HCl was forcibly administered before administering the capsules of the present invention. Immediately thereafter, the capsules were forcibly administered orally to the normal gastric acidity model animals, and the expired air was collected over time.

On the other hand, to provide a hypoacidic condition to the beagle dogs, the expired air was collected and an H₂ antagonist (10 mg of famotidine hydrochloride) was forcibly administered before administering the capsules of the present invention. After one hour after the H₂ antagonist administration, the capsules were forcibly administered orally to the hypoacidity model animals, and the expired air was collected over time.

### (3) Measurement of gastric pH

Using GC-MS (ABCA-G, manufactured by Europa Scientific), the ¹³CO₂/¹²CO₂ concentration ratios (the δ¹³C values) in the expired air were measured before administering the capsules of the present invention and at each collection time after the administration, and the Δ value (‰) ([the δ¹³C value after administering the capsules] - [the δ¹³C value before the administration]) was calculated from the difference in the δ¹³C value between each collection time after administering the capsules and before the administration.

### (4) Results

Fig. 1 presents the average patterns of excretion in the expired air of the normal gastric acidity model animals (n=4, -■-) and the hypoacidity model animals (n=4, -◆-), by plotting the time lapsed after the administration (hours) on the abscissa and the Δ value (‰) on the ordinate. The figure reveals that in the hypoacidity model animals, the time to reach maximum Δ value (‰) (Tmax) was 1 hour, whereas in the normal gastric acidity model animals, the Tmax was 2.7 hours, indicating that the ¹³CO₂ excretion behavior in the expired air greatly- differs between the hypoacidity model animals and the normal acidity model animal. These results demonstrate that the preparation for measuring gastric pH of the present invention makes it possible to monitor gastric pH, and in particular to identify and assess, with significant differences, the hypoacidic tendency and normal acidic tendency, and hyperacidic tendency as well, in the stomach.

### Example 2

### (1) Production of a preparation for measuring gastric pH (capsules)

NaH¹³CO₃ (100 mg) was encapsulated in an enteric capsule base (60 mg; Capsule No. 1 defined in The Japanese Pharmacopoeia, hydroxypropylmethylcellulose phthalate, tradename: HP-55, manufactured by Shin-Etsu Chemical Co., Ltd. (dissolves at pH 5.5 or higher)), to produce an enteric preparation for measuring gastric pH (capsules 1). Separately, NaH¹³CO₃ (100 mg) was encapsulated in a gastro-soluble capsule base (60 mg; Capsule No. 1 defined in The Japanese Pharmacopoeia, aminoacryl methacrylate copolymer E, tradename: Eudragit E100, manufactured by Rohm Pharma (dissolves at pH 5 or lower)), to produce a gastro-soluble preparation for measuring gastric pH (capsules 2). The following experiment was carried out using these preparations.

### (2) Animal experiment

In the experiment, four fasted beagle dogs were used without adjustment of gastric pH. Capsules 1 or 2 produced above were administered to the beagle dogs (n=4), and the ¹³CO₂ excretion behavior in the expired air was measured to evaluate the gastric pH in the four beagle dogs.

The detailed procedures of the experiment were as follows: The expired air of the beagle dogs was collected, capsules 1 were forcibly administered orally, and then the expired air was collected over time. After one week from the administration, the expired air was collected, capsules 2 were forcibly administered orally, and then the expired air was collected over time.

### (3) Measurement of gastric pH

Using GC-MS (ABCA-G, manufactured by Europa Scientific), the ¹³CO₂/¹²CO₂ concentration ratios (the δ¹³C values) in the expired air were measured before administering the capsules and at each collection time after the administration, and the Δ value (‰) ([the δ¹³C value after administering the capsules] - [the δ¹³C value before the administration]) was calculated from the difference between the δ¹³C value at each collection time after administering the capsules and the δ¹³C value before the administration.

### (4) Results

Fig. 2 presents the patterns of excretion in the expired air of the four beagle dogs after administering capsules 1, and Fig. 3 shows the patterns of excretion in the expired air of the four beagle dogs after administering capsules 2. Fig. 2 shows that, when the enteric capsules were administered, the time to reach maximum Δ value (‰) (Tmax) in beagle dogs No. 2 (-■-), No. 3 (-▲-), and No. 4 (-●-) was as little as 1 to 1.3 hours, whereas the T max in beagle dog No. 1 (-◆-) was as much as 2.3 hours. This indicates that beagle dogs Nos. 2, 3, and 4 had a high gastric pH (the hypoacidic tendency), and beagle dog No. 1 had a low gastric pH.

On the other hand, Fig. 3 shows that, when capsules 2 were administered, a reaction was detected only in the expired air of beagle dog No. 1 (-◆-), and was not detected in the expired air of beagle dogs Nos. 2, 3, and 4. This indicates that beagle dog No. 1 had a low gastric pH and beagle dogs Nos. 2, 3, and 4 had a high gastric pH (the hypoacidity tendency), supporting the results obtained by administering capsules 1.

The above results demonstrate that the preparation for measuring gastric pH of the present invention makes it possible to monitor gastric pH, and to identify and evaluate the hypoacidic tendency and normal acidic tendency, and hyperacidic tendency as well.

### Formulation Example 1 Capsules

| <Labeled compound-containing material> | |
|---|---|
| Sodium hydrogencarbonate (NaH¹³CO₃) | 100 mg |
| Magnesium stearate | 1 mg |

| <Gastro-soluble capsules> | |
|---|---|
| Aminoacryl methacrylate copolymer E (Eudragit E100, manufactured by Rohm Pharma) | 50 mg |

A preparation for measuring gastric pH according to the present invention was produced by filling the gastro-soluble capsules with the labeled compound-containing material.

### Formulation Example 2 Tablets

| <Labeled compound-containing material> | |
|---|---|
| Sodium hydrogencarbonate (NaH¹³CO₃) | 100 mg |
| Lactose | 40 mg |
| Magnesium stearate | 2 mg |

| <Gastro-soluble base (coating)> | |
|---|---|
| Aminoacryl methacrylate copolymer E | |
| (Eudragit E100, manufactured by Rohm Pharma) | 40 mg |

A preparation for measuring gastric pH according to the present invention was produced by covering the labeled compound-containing material as the core tablet with the gastro-soluble base (coating) in a routine manner.

### Formulation Example 3 Granules

| <Labeled compound-containing material> | |
|---|---|
| Sodium hydrogencarbonate (NaH¹³CO₃) | 100 mg |
| Purified sucrose (powder) | 150 mg |
| Cornstarch | 100 mg |
| Crystalline cellulose (Avicel pH-301: Asahi Kasei Corporation) | 100 mg |
| Carmellose calcium (ECG-505: Nichirin Chemical Ind. Co., Ltd.) | 50 mg |
| Hydroxypropyl cellulose (HPC-L: Shin-Etsu Chemical Co., Ltd.) | 7.5 mg |
| Purified water | 75 mg |
| Anhydrous ethanol | 67.5 mg |
| Total solids | 507.5 m |

| <Enteric base: Coating liquid> | |
|---|---|
| Hydroxypropylmethylcellulose phthalate (HP-55: Shin-Etsu Chemical Co., Ltd.) | 6.0 mg |
| Talc | 1.8 mg |
| Anhydrous ethanol | 73.8 mg |
| Purified water | 18.4 mg |
| Total | 100.0 mg |

Using labeled sodium hydrogencarbonate (NaH¹³CO₃) as the main ingredient, the ingredients listed in <Labeled compound-containing material> were blended, kneaded, and granulated in a routine manner. The obtained core granules were sprayed and covered with the coating liquid of the above formula to produce granules comprising 100 parts by weight of core granules covered with 40 parts by weight of hydroxypropylmethylcellulose phthalate as an enteric base (coating). The granules had an average particle diameter of 1000 to 1400 µm.

### INDUSTRIAL APPLICABILITY

Using the preparation for measuring gastric pH of the present invention, it is possible to easily and accurately measure and assess the gastric pH or gastric pH tendency (the hyperacidic, normal, hypoacidic, or anacidic tendency) in a human or animal. In particular, the preparation for measuring gastric pH produced using a labeled compound to be excreted as labeled carbon dioxide in the expired air makes it possible to easily measure gastric pH or its tendency by an expiration test, without placing any physical or mental burden on the subject.

Further, with the preparation for measuring gastric pH of the present invention or the method for measuring gastric pH using the preparation, the presence or absence of a decrease or increase in the subject's gastric pH can be easily and accurately diagnosed and assessed. Also, it is possible to directly and easily diagnose a disease relating to gastric pH, and to directly and easily assess and evaluate the therapeutic effects for a disease relating to gastric pH and the efficacies of a medicine relating to gastric pH, by using the preparation and method of the present invention.

## Claims

1. A preparation for measuring gastric pH comprising a composition containing an isotope-labeled compound, and a pH-dependent soluble base covering the composition.

2. A preparation according to claim 1, wherein the isotope is at least one member selected from the group consisting of ¹³C, ¹⁴C, ¹⁵N, and ¹⁸O.

3. A preparation according to claim 1, wherein the isotope-labeled compound is an alkali metal carbonate, an alkaline earth metal carbonate, ammonium carbonate, an alkali metal hydrogencarbonate, or ammonium hydrogencarbonate.

4. A preparation according to claim 1, wherein the isotope-labeled compound is at least one member selected from the group consisting of sodium carbonate, potassium carbonate, calcium carbonate, magnesium carbonate, ammonium carbonate, potassium hydrogencarbonate, sodium hydrogencarbonate, and ammonium hydrogencarbonate.

5. A preparation according to claim 1, wherein the composition containing an isotope-labeled compound further contains at least one acid compound selected from the group consisting of citric acid, tartaric acid, and malic acid.

6. A preparation according to claim 1, wherein the isotope-labeled compound is at least one member selected from the group consisting of amino acids, proteins, organic acids, salts of organic acid, saccharides, and lipids.

7. A preparation according to claim 1, wherein the pH-dependent soluble base is an enteric base or gastro-soluble base.

8. A preparation according to claim 1, wherein the pH-dependent soluble base is at least one gastro-soluble base selected from the group consisting of methyl methacrylate-butyl methacrylate-dimethylaminoethyl methacrylate copolymers, and polyvinyl acetal diethyl acetate.

9. A preparation according to claim 1, wherein the pH-dependent soluble base is at least one enteric base selected from the group consisting of hydroxypropylmethylcellulose phthalate, methacrylic acid-methyl methacrylate copolymers, hydroxypropylmethylcellulose acetate succinate, carboxymethylethylcellulose, hydroxypropylmethylcellulose phthalate, and cellulose acetate phthalate.

10. A method for measuring gastric pH using a preparation according to claim 1.

11. A method for measuring gastric pH, comprising orally administering a preparation according to claim 1 to a subject, and measuring the behavior of a labeled compound excreted from the body.

12. A method for measuring gastric pH, comprising orally administering a preparation according to claim 2 to a subject, and measuring the amount or behavior of labeled CO₂ excreted in the expired air.

13. A method according to claim 10, wherein a preparation as defined in claim 1 comprising an enteric base as the pH-dependent soluble base is used in combination with a preparation as defined in claim 1 comprising a gastro-soluble base as the pH-dependent soluble base.

14. A method for measuring gastric pH in a subject, comprising orally administering a preparation as defined in claim 1 to a subject who is suspected of having a decreased or increased gastric pH, and comparing the behavior of a labeled compound excreted from the body after a prescribed period of time with a standard control.

15. Use of a preparation as defined in claim 1 in a method for measuring gastric pH.

16. Use of an isotope-labeled compound covered with a pH-dependent soluble base, for producing a preparation for measuring gastric pH for use in a method for measuring gastric pH.

17. Use according to claim 16, wherein the isotope-labeled compound is an alkali metal carbonate, an alkaline earth metal carbonate, ammonium carbonate, an alkali metal hydrogencarbonate, or ammonium hydrogencarbonate.
